(19) Europäisches Patentamt
European Patent Office
Office européen des brevets

(11) Numéro de publication: **0 162 036 B1**

# (12) FASCICULE DE BREVET EUROPEEN

(45) Date de publication du fascicule du brevet: **16.08.89**

(51) Int. Cl.⁴: **C 07 D 207/27**, A 61 K 31/40

(21) Numéro de dépôt: **85870069.3**

(22) Date de dépôt: **14.05.85**

(54) **(S)-alpha-éthyl-2-oxo-1-pyrrolidineacétamide.**

(30) Priorité: **15.05.84 GB 8412357**

(43) Date de publication de la demande: **21.11.85 Bulletin 85/47**

(45) Mention de la délivrance du brevet: **16.08.89 Bulletin 89/33**

(84) Etats contractants désignés: **AT BE CH DE FR GB IT LI LU NL SE**

(56) Documents cités:
**FR-A- 2 081 508**
**FR-A- 2 368 275**

(73) Titulaire: **U C B, S.A., 326, Avenue Louise, B-1050 Bruxelles (BE)**

(72) Inventeur: **Gobert, Jean, 120, rue du Cornet, B-1040 Bruxelles (BE)**
Inventeur: **Geerts, Jean-Pierre, 12, Habaru, B-6737 Leglise (BE)**
Inventeur: **Bodson, Guy, 248, rue de la Chevratte, B-6739 Bellefontaine (Tintigny) (BE)**

(74) Mandataire: **Dusseldorp, Raymond et al, U.C.B. S.A. Département D.T.B. 326, avenue Louise, Bte 7, B-1050 Bruxelles (BE)**

EP 0 162 036 B1

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

## Description

La présente invention se rapporte à un composé nouveau, le (S)-alpha-éthyl-2-oxo-1-pyrrolidineacétamide ainsi qu'à des procédés pour le préparer. Elle concerne également les compositions thérapeutiques renfermant ledit composé.

Dans le brevet britannique no 1 309 692 au nom de la demanderesse, on décrit l'alpha-éthyl-2-oxo-1-pyrrolidineacétamide (point de fusion 122°C) et on indique que les substances de ce type sont utilisables dans le domaine thérapeutique, par exemple pour le traitement du mal de mouvement, des hyperkinésies, des hypertonies et de l'épilepsie.

D'autre part, on y mentionne aussi que ces composés peuvent trouver une application dans le domaine des troubles de la mémoire dans des conditions normales ou pathologiques.

En outre, il est connu également que l'alpha-éthyl-2-oxo-1-pyrrolidineacétamide possède une activité de protection envers les agressions du système nerveux central causées par des hypoxies, de l'ischémie cérébrale, etc. (Pharmazie, 37/11, (1982), 753–765).

Poursuivant ses travaux de recherche dans ce domaine, la demanderesse a préparé et isolé l'énantiomère lévogyre de l'alpha-éthyl-2-oxo-1-pyrrolidineacétamide et elle a trouvé que ce composé se distingue de manière tout à fait imprévue de la forme racémique connue

1) par une activité de protection envers l'hypoxie (anti-hypoxie) 10 fois plus élevée et
2) par une activité de protection envers l'ischémie cérébrale (anti-ischémie) 4 fois supérieure.

De cet ensemble imprévisible de propriétés, il résulte que l'énantiomère lévogyre de l'alpha-éthyl-2-oxo-1-pyrrolidineacétamide convient mieux pour le traitement et la prévention des agressions du type hypoxique et ischémique de système nerveux central. La contribution importante du phénomène hypoxique dans certaines pathologies du système nerveux central laisse présager un effet thérapeutique de ce produit dans le traitement des suites d'accidents vasculaires cérébraux et de traumatismes crâniens, des séquelles du processus de vieillissement ou des insuffisances circulatoires du système nerveux central résultant d'accidents cérébroischémiques ou d'hypoxie survenant p.ex. lors de la naissance. Par extension, il est permis d'envisager l'utilisation de ce produit dans les affections du type hypoxique d'autres organes ou tissus: cœur et reins.

C'est pourquoi la présente invention a pour objet l'énantiomère lévogyre de l'alpha-éthyl-2-oxo-1-pyrrolidineacétamide qui possède la configuration absolue S, ledit composé étant substantiellement exempt de l'énantiomère dextrogyre ayant la configuration absolue R.

Le (S)-alpha-éthyl-2-oxo-1-pyrrolidineacétamide conforme à la présente invention ne peut pas être obtenu directement à partir de la forme racémique par séparation des deux énantiomères. Il peut être préparé par l'un ou l'autre des procédés suivants:

a) on fait réagir l'acide (S)-alpha-éthyl-2-oxo-1-pyrrolidineacétique successivement avec (1) un halogénoformiate d'alkyle de formule HalCOOZ dans laquelle Hal représente un atome d'halogène et Z un radical alkyle ayant 1 à 4 atomes de carbone et (2) de l'ammoniac. L'halogénoformiate d'alkyle est de préférence du chloroformiate d'éthyle.

Cette réaction est généralement effectuée dans le dichlorométhane, à une température comprise entre −10 et −60 °C.

L'acide (S)-alpha-éthyl-2-oxo-1-pyrrolidineacétamide, utilisé dans cette réaction, peut être obtenu à partir de l'acide (±)-alpha-éthyl-2-oxo-1-pyrrolidineacétique racémique, par résolution chimique selon des méthodes connues en soi, par exemple en formant un sel de cet acide avec une base optiquement active et en isolant le sel formé avec l'acide (S)-alpha-éthyl-2-oxo-1-pyrrolidineacétique, par cristallisations successives dans un solvant approprié (p.ex. le benzène).

A titre d'exemples de bases optiquement actives pouvant être utilisées pour cette résolution, on peut citer des alcaloïdes comme la brucine, la quinine, la strychnine, la quinidine, la cinchonidine et des amines comme l'alpha-méthyl-benzylamine et la déhydroabiétylamine (cfr. S. H. Wilen et al., Tetrahedron, 33, (1977), 2725–2736). Des résultats particulièrement favorables sont obtenus en utilisant l'alpha-méthyl-benzylamine et la déhydroabiétylamine.

Quant à l'acide (±)-alpha-éthyl-2-oxo-1-pyrrolidineacétique racémique utilisé comme matière première, il peut être obtenu par saponification des esters alkyles correspondants, dont la synthèse a été décrite dans le brevet britannique no 1 309 692.

b) on cyclise un (S)-2-amino-butanamide de formule

$$X{-}CH_2CH_2{-}Y{-}NHCH(C_2H_5)CONH_2 \qquad (A)$$

dans laquelle

X représente un radical ZOOC– ou $HalCH_2$–, Z étant un radical alkyle ayant 1 à 4 atomes de carbone et Hal un atome d'halogène, de préférence le chlore ou le brome, et

Y représente un radical $-CH_2-$ ou –CO–,

avec la restriction que lorsque X représente un radical ZOOC–, Y est un radical $-CH_2-$ et lorsque X représente un radical $HalCH_2$–, Y est un radical –CO–.

La cyclisation du (S)-2-amino-butanamide de formule A est effectuée dans un solvant inerte, tel que le toluène ou le dichlorométhane, à une température comprise entre 0 °C et la température d'ébullition du solvant. Cette cyclisation s'opère en présence d'une substance basique, en tant que catalyseur. Ce catalyseur est la 2-hydroxypyridine, lorsque le composé de formule A est un ester (X = ZOOC–) et le bromure de tétrabutylammonium lorsque le composé de formule A est un halogénure (X = $HalCH_2$–).

Lorsque X représente un radical ZOOC– et Y est und radical $-CH_2-$, le composé de formule A est un (S)-4-[[l-(aminocarbonyl)propyl]amino]-butyrate d'alkyle de formule $ZOOCCH_2CH_2CH_2NHCH(C_2H_5)CONH_2$, dans laquelle Z a la signification donnée plus haut. Ce dernier peut être préparé par condensation de (S)-2-amino-butanamide avec un 4-halogénobutyrate d'alkyle de formule $ZOOCCH_2CH_2CH_2$–Hal dans laquelle Z a la signification donnée plus et Hal est un atome d'halogène. Lorsque X représente un radical $HalCH_2$– et que Y est alors un radical -CO–, le composé de formule A est un (S)-N-[l-(aminocarbonyl)propyl]-4-halogénobutanamide de formule $HalCH_2CH_2CH_2CONHCH(C_2H_5)CONH_2$, dans laquelle Hal a la signification donnée plus haut. Ce dernier peut être préparé par condensation de (S)-2-aminobutanamide avec un halogénure de 4-halogénobutyryle de formule $HalCH_2CH_2CH_2COHal$ dans laquelle Hal est un atome d'halogène.

La réaction entre le (S)-2-amino-butanamide d'une part et le 4-halogénobutyrate d'alkyle ou l'halogénure de 4-halogénobutyryle d'autre part, est généralement effectuée dans un solvant inerte, tel que le benzène, le toluène, le dichlorométhane ou l'acétonitrile, à une température comprise entre −5 et +100 °C et en présence d'un accepteur d'acide tel qu'une base organique tertiaire (p. ex. la triéthylamine) ou une base minérale (p. ex. le carbonate ou l'hydroxyde de potassium ou de sodium).

Lorsque X représente un radical $HalCH_2$– et Y un radical -CO–, il n'est pas indispensable d'isoler le composé de formule A obtenu à partir des matières premières citées plus haut. En effet, le composé de formule A, obtenu in situ, peut être cyclisé directement en le (S)-alpha-éthyl-2-oxo-1-pyrrolidineacétamide conforme à la présente invention (voir l'exemple 4 ci-après).

Le (S)-2-amino-butanamide utilisé comme matière première, peut être obtenu à partir de l'acide (S)-2-amino-butyrique par ammonolyse de l'ester méthylique correspondant selon la méthode décrite par K. Folkers et al. dans J. Med. Chem. 14, (6), (1971), 484–487.

Les exemples qui suivent illustrent l'invention sans la limiter. Das ces exemples, la pureté optique des produits obtenus a été vérifiée par la détermination calorimétrique des enthalpies différentielles (C. Fouquey et J. Jacques, Tetrahedron, 23, 1967, 4009–19).

Exemple 1

a) Préparation du sel de (R)-alpha-méthyl-benzylamine de l'acide (S)-alpha-éthyl-2-oxo-1-pyrrolidineacétique.

Dans un réacteur de 50 litres, on met en suspension 8,7 kg (50,8 moles) d'acide (±)-alpha-éthyl-2-oxo-1-pyrrolidineacétique racémique dans 21,5 litres de benzène anhydre. A cette suspension, on ajoute progressivement une solution contenant 3,08 kg (25,45 moles) de (R)-(+)-alpha-méthyl-benzylamine et 2,575 kg (25,49 moles) de triéthylamine dans 2,4 litres de benzène anhydre. On porte ensuite le mélange à reflux jusqu'à dissolution complète. On refroidit et on laisse cristalliser pendant quelques heures. On obtient ainsi 5,73 kg de sel de (R)-alpha-méthyl-benzylamine de l'acide (S)-alpha-éthyl-2-oxo-1-pyrrolidineacétique.
P. F.: 148–151 °C. Rendement: 77,1 %.
Ce sel peut être purifié par chauffage à reflux pendant 4 heures dans 48,3 litres de benzène. On refroidit et on filtre. On recueille ainsi 5,040 kg du sel souhaité.
P. F.: 152–153,5 °C. Rendement: 67,85%.

b) Préparation de l'acide (S)-alpha-éthyl-2-oxo-1-pyrrolidineacétique.
On dissout 5,04 kg de sel obtenu en a) ci-dessus dans 9 litres d'eau. On y ajoute lentement 710 g d'une solution d'hydroxyde de sodium à 30%. Le pH atteint 12,6 et la température ne dépasse pas 25 °C. On agite la solution encore pendant 20 minutes et on extrait l'alpha-méthyl-benzylamine libérée en plusieurs fois par un volume total de 18 litres de benzène.

On acidifie ensuite la phase aqueuse jusqu'à une valeur de pH de 1,1 par addition de 3,2 litres d'acide chlorhydrique 6N. Le précipité formé est filtré, lavé à l'eau et séché.

On extrait le filtrat en plusieurs fois par un volume total de 50 litres de dichlorométhane. On sèche le phase organique sur sulfate de sodium, on la filtre et on l'évapore à sec sous pression réduite.

Le résidu obtenu après évaporation et le précipité isolé précédemment sont dissous ensemble à chaud dans 14 litres de dichlorométhane. Le dichlorométhane est distillé et remplacé au fur et à mesure par 14 litres de toluène dans lequel le produit cristallise. On refroidit à la température ambiante et on filtre les cristaux. On obtient ainsi 2,780 kg d'acide (S)-alpha-éthyl-2-oxo-1-pyrrolidineacétique.
P.F.: 125,9 °C. $[alpha]_D^{20}$ = −26,4 °C (c = 1, acétone). Rendement: 94,5%.

c) Préparation du (S)-alpha-éthyl-2-oxo-1-pyrrolidineacétamide.
On met en suspension 34,2 g (0,2 mole) de l'acide (S)-alpha-éthyl-2-oxo-1-pyrrolidineacétique dans 225 ml de dichlorométhane, refroidi à −30 °C. On y ajoute goutte à goutte 24,3 g (0,24 mole) de triéthylamine en 15 minutes. Le mélange réactionnel est alors refroidi à −40 °C et on y ajoute 24,3 g (0,224 mole) de chloroformiate d'éthyle en 12 minutes. Ensuite on fait passer un courant d'ammoniac pendant 4 heures et demie. On laisse revenir à la température ambiante et on élimine les sels d'ammonium formés par filtration et lavage avec du dichlorométhane. On distille le solvant sous pression réduite. Le résidu solide ainsi obtenu est mis en dispersion dans 55 ml de toluène et agité pendant 30 minutes, puis filtré et recristallisé dans 280 ml d'acétate d'éthyle en

présence de 9 g tamis moléculaire 0,4 nm en poudre.

On obtient 24,6 g de (S)-alpha-éthyl-2-oxo-1-pyrrolidineacétamide.

P.F: 115-118°C $[alpha]_D^{25}$ = -89,7°C (c = 1, acétone). Rendement: 72,3%.

Analyse pour $C_8H_{14}N_2O_2$ en %

| | | | |
|---|---|---|---|
| calculé: | C 56,45 | H 8,29 | N 16,46 |
| trouvé: | C 56,71 | H 8,22 | N 16,48 |

L'acide (±)-alpha-éthyl-2-oxo-1-pyrrolidineacétique racémique utilisé dans cette synthèse a été préparé de la manière décrite ci-dessous. Dans un ballon de 20 litres contenant 3,65 kg (18,34 moles) de (±)-alpha-éthyl-2-oxo-1-pyrrolidineacétate d'éthyle, on ajoute en deux heures, à une température ne dépassant pas 60°C, une solution contenant 788 g (19,7 moles) d'hydroxyde de sodium dans 4,35 litres d'eau. Lorsque cette addition est terminée, on porte la température du mélange à 80°C et on distille l'alcool formé jusqu'à ce que la température dans le milieu atteigne 100°C. On refroidit à 0°C et on ajoute en 2 heures et demie, 1,66 litre (19,60 moles) d'acide chlorhydrique 12N. On filtre le précipité, on le lave avec deux litres de toluène et on le recristallise dans de l'alcool isopropylique. On obtient ainsi 2,447 kg d'acide (±)-alpha-éthyl-2-oxo-1-pyrrolidineacétique racémique, fondant à 155-156°C. Rendement: 78%.

Analyse pour $C_8H_{13}NO_3$ en %

| | | | |
|---|---|---|---|
| calculé: | C 56,12 | H 7,65 | N 8,18 |
| trouvé: | C 55,82 | H 8,10 | 7,97 |

Exemple 2

a) Préparation du (S)-4-[[1-(aminocarbonyl)propyl]amino]-butyrate d'éthyle.

A une suspension de 47,75 g (0,345 mole) de chlorhydrate de (S)-2-amino-butanamide ($[alpha]_D^{25}$: +26,1°C; c = 1, méthanol) dans 400 ml de toluène, on ajoute 143,6 ml (1,035 mole) de triéthylamine. On porte le mélange à 80°C et on y introduit goutte à goutte 67,2 g (0,345 mole) de 4-bromobutyrate d'éthyle.

On maintient le milieu réactionnel à 80°C pendant 10 heures, puis on filtre à chaud pour éliminer les sels de triéthylamine. On évapore le filtrat sous pression réduite et on obtient 59 g d'un résidu huileux constitué essentiellement de produit de monoalkylation et contenant un peu de dérivé dialkylé.

Le produit, obtenu à l'état brut, a été utilisé tel quel, sans autre purification, dans la préparation du (S)-alpha-éthyl-2-oxo-1-pyrrolidineacétamide par cyclisation.

b) Préparation du (S)-alpha-éthyl-2-oxo-1-pyrrolidineacétamide.

On met 54 g du produit brut obtenu en a) ci-dessus, en solution dans 125 ml de toluène, en présence de 2 g de 2-hydroxypyridine. On maintient le mélange à 100°C pendant 12 heures.

On filtre à chaud l'insoluble, puis on évapore le filtrat sous pression réduite.

On purifie le résidu par chromatographie sur une colonne de 1,1 kg de silice (diamètre de la colonne: 5 cm; éluant: un mélange d'acétate d'éthyle, de méthanol et d'ammoniaque concentré dans des proportions en volume de 85:12:3).

Le produit isolé est recristallisé dans 50 ml d'acétate d'éthyle. On recueille ainsi 17,5 g de (S)-alpha-éthyl-2-oxo-1-pyrrolidineacétamide.

P.F.: 117°C $[alpha]_D^{25}$: -90,0°C (c = 1, acétone). Rendement: 41%.

Exemple 3

a) Préparation du (S)-N-[1-(aminocarbonyl)propyl]-4-chloro-butanamide.

On mélange 345,6 g (2,5 moles) de carbonate de potassium broyé et 138,5 g (1 mole) de chlorhydrate de (S)-2-amino-butanamide dans 2,5 litres d'acétonitrile. On refroidit le mélange réactionnel vers 0°C et on y introduit goutte à goutte, une solution de 129,2 g (1,2 mole) de chlorure de 4-chlorobutyryle dans 500 ml d'acétonitrile. Après l'addition, on laisse le mélange réactionnel revenir à la température ambiante; on élimine l'insoluble par filtration et on évapore le filtrat sous pression réduite. Le résidu brut obtenu est agité pendant 30 minutes dans 1,2 litre d'éther anhydre à une température comprise entre 5 et 10°C. On filtre le présipité, on le lave par 2 fois 225 ml d'éther et on le sèche sous vide. On obtient ainsi 162,7 g de (S)-N-[1-(aminocarbonyl)propyl]-4-chloro-butanamide.

P.F.: 118-123°C $[alpha]_D^{25}$ : -18°C (c=1, méthanol). Rendement: 78,7%.

Le produit brut ainsi obtenu convient bien pour le stade suivant de cyclisation. Il peut cependant être purifié par agitation pendant une heure dans de l'acétate d'éthyle anhydre.

P.F.: 120-122°C $[alpha]_D^{25}$: -22,2°C (c = 1, méthanol).

b) Préparation du (S)-alpha-éthyl-2-oxo-1-pyrrolidineacétamide.

A 0°C et sous atmosphère d'azote, on mélange dans 45 ml de dichlorométhane, 6,2 g (0,03 mole) de (S)-N-[1-(aminocarbonyl)propyl]-4-chlorobutanamide et 0,484 g (0,0015 mole) de bromure de tétrabutylammonium. Sans dépasser +2°C dans le ménage réactionnel, on ajoute en 30 minutes 2,02 g (0,036 mole) d'hydroxyde de potassium pulvérulent. Le mélange est ensuite agité pendant une heure, puis on y ajoute encore 0,1 g (0,0018 mole) d'hydroxyde de potassium broyé et on agite encore pendant 30 minutes à 0°C. On laisse revenir à température ambiante. On élimine l'insoluble par filtration et on concentre le filtrat sous pression réduite. Le résidu obtenu est recristallisé dans 40 ml d'acétate d'éthyle en présence de 1,9 g de tamis moléculaire 0,4 nm, qui est éliminé par filtration à chaud. On obtient ainsi 3,10 g de (S)-alpha-éthyl-2-oxo-1-pyrrolidineacétamide.

P.F.: 116,7°C $[alpha]_D^{25}$ : -90,1°C (c = 1, acétone). Rendement: 60,7%.

Exemple 4

Préparation du (S)-alpha-éthyl-2-oxo-1-pyrrolidineacétamide.

Cet exemple illustre une variante du procédé de l'exemple 3, dans laquelle le 4-chloro-butanamide intermédiaire obtenu in situ, n'est pas isolé.

A une suspension de 69,25 g (0,5 mole) de chlorhydrate de (S)-2-amino-butanamide dans 600 ml de dichlorométhane, on ajoute à la température ambiante 84 g de sulfate de sodium anhydre. On refroidit à 0°c et on ajoute 115 g d'hydroxyde de potassium broyé, puis 8,1 g (0,025 mole) de bromure de tétrabutylammonium dissous dans 100 ml de dichlorométhane. Sous bonne agitation, on ajoute goutte à goutte, à 0°C, une solution de 77,5 g de chlorure de 4-chlorobutyle dans 100 ml de dichlorométhane. Après 5 heures de réaction, on ajoute encore 29 g d'hydroxyde de potassium broyé. Deux heures plus tard, on filtre le mélange réactionnel sur Hyflo-cel (Hyflo-cel est une marque commerciale) et on évapore le filtrat sous pression réduite. On met le résidu (93,5 g) en dispersion dans 130 ml de toluène chaud pendant 45 minutes. On filtre et on évapore sous pression réduite. On dissout le résidu (71,3 g) à chaud dans 380 ml d'acétate d'éthyle, auquel on ajoute 23 g de tamis moléculaire 0,4 nm en poudre. On porte ce mélange à reflux et on filtre à chaud. Après refroidissement du filtrat, le produit souhaité cristallise. On recueille ainsi 63 g de (S)-alpha-éthyl-2-oxo-1-pyrrolidineacétamide.

P.F.: 117°C $[alpha]_D^{25}$ : -91,3°C (c = 1, acétone).

Rendement: 74,1%

Essais pharmacologiques.

L'alpha-éthyl-2-oxo-1-pyrrolidineacétamide racémique (produit A) et le (S)-alpha-éthyl-2-oxo-1-pyrrolidineacétamide (produit B) de la présente invention ont été soumis à des tests pharmacologiques.

I. Protection envers l'hypoxie (souris).

a. Principe (C. Giurgea et F. Mouravieff-Lesuisse; Proc. Xth Intern. Congr. of the Coll. Intern. Neuro.-psych. - Pergamon Press, Oxford and New York, 1978, p. 1623-1631).

Le principe de ce test consiste à mesurer les possibilités de survie de l'organisme soumit à une atmosphère progressivement appauvrie en oxygène. Etant donné la sensibilité particulière du système nerveux à ce type d'agression, les résultats obtenus dans ce test peuvent s'interpréter comme une mesure de résistance du système nerveux. Les produits augmentant la résistance des animaux conviennent par conséquent pour le traitement et la prévention des agressions du type hypoxique du système nerveux central.

b. Méthode

L'appareil consiste en une enceinte étanche et transparente mesurant 37 cm de haut, 39 cm de profondeur et 97 cm de largeur. Cette cage de 140 litres est munie de 60 compartiments transparents de 6 × 10 × 10 cm permettant d'accueillir séparément 60 souris.

Un ventilateur y assure le brassage de l'atmosphère qui circule parmi les compartiments au travers d'un plancher grillagé. L'enceinte est munie d'un dispositif d'admission d'azote à débit constant et d'une ouverture communiquant avec l'air ambiant.

Les animaux sont des souris (souche NMRI) mâles de 20 à 22 g. Celles-ci sont mises à jeun la vieille du test. L'expérience se pratique le lendemain et simultanément sur trois groupes de 20 souris: un groupe témoin reçoit de l'eau (25 ml/kg) par voie orale et les deux autres groupes reçoivent chacun per os un produit à tester.

Vingt-cinq minutes après l'administration, les animaux sont répartis au hasard parmi les compartiments de manière à ce qu'aucun des trois groupes d'animaux ne soit localisé en un endroit préférentiel de la cage.

Trente minutes après l'administration, la cage est fermée et l'azote y est admis à un débit constant (7,750 litres d'azote technique par minute) pendant environ 37 minutes; l'atmosphère contient alors 3,7% d'oxygène.

La cage reste fermée jusqu'au moment critique où l'on n'observe plus que trois survivants parmi les vingt animaux témoins. A ce moment, la cage est ouverte l'air ambiant y est admis. Quelques instants plus tard, les survivants sont dénombrés parmi chaque groupe d'animaux.

Pour chaque dose de produit à tester, les expériences sont répétées une ou deux fois, et les résultats sont globalisés pour obtenir un minimum de 40 (ou 60) animaux traités par dose et 40 (ou 60) animaux témoins correspondants.

Pour chaque dose de produit testé, le nombre d'animaux survivants parmi ceux traités par le produit est comparé au nombre d'animaux survivants parmi les animaux témoins. La différence entre ces nombres exprime l'activité de protection du produit envers l'hypoxie causée par la privation d'oxygène. La signification statistique (P) de cette différence est évaluée par le test de Fischer-Yates.

c. Résultats

Dans le tableau I ci-dessous, on donne les résultats obtenus pour des doses croissantes des produits A et B.

Tableau I

| Produit testé | Dose per os en mmol/kg | Nombre d'animaux survivants | | P |
|---|---|---|---|---|
| | | témoins | traités | |
| A | 0,032 | 12/60 | 16/60 | NS |
| | 0,1 | 8/60 | 7/60 | NS |
| | 0,16 | 12/60 | 12/60 | NS |
| | 0,32 | 10/60 | 30/60 | <0,001 |
| B | 0,016 | 5/40 | 11/40 | NS |
| | 0,032 | 8/40 | 17/40 | <0,6 |
| | 0,1 | 6/40 | 19/40 | <0,005 |
| | 0,16 | 6/40 | 19/40 | <0,005 |
| | 0,32 | 5/40 | 17/40 | <0,01 |

NS = différence non significative.

d. Conclusions

Dans ce test, l'énantiomère lévogyre de l'invention (produit B) augmente la survie des animaux privés d'oxygènes à partir d'une dose de 0,032 mmol/kg. Le racémate (produit A) exerce une activité similaire seulement à partir de 0,32 mmol/kg (Ire dose efficace: et est donc 10 fois moins actif que l'énantiomère lévogyre.

II. Protection envers l'ischémie cérébrale (rats).

a. Principe (C. Giurgea et F. Mouravieff-Lesuisse; loc. cit. sous I. a.).

Des contrôles électroencéphalographiques ont montré que la ligature des deux carotides communes chez le rat, provoque une véritable ischémie cérébrale: le tracé de l'électroencephalogramme s'applatit et devient même isoélectrique (silence électrique).

b. Méthode

Des rats Wistar mâles d'un poids compris entre 250 et 350 g sont anesthésiés au pentobarbital, administré par voie intrapéritonéale à la dose de 50 mg/kg (0,5 ml/100 g).

Immédiatement après l'anesthésie, on administre aux animaux, par voie intrapéritonéale, à raison de 0,5 ml/100 g, soit le produit à tester dissous dans une solution de chlorure de sodium isotonique (animaux traités), soit uniquement une solution de chlorure de sodium isotonique ou placebo (animaux témoins).

Environ 20 minutes plus tard, les deux carotides communes sont dénudées et environ dix minutes après, elles sont ligaturées simultanément. Cette opération se fait simultanément sur les animaux témoins et sur les animaux traités.

Une heure après l'administration du produit à tester ou du placebo, on administre à nouveau par voie intrapéritonéale, une même dose, soit du produit à tester (aux animaux traités), soit du placebo (aux animaux témoins).

Cinq heures après la première administration, on injecte une troisième fois une dose, soit du produit à tester (aux animaux traités survivants), soit du placebo (aux animaux témoins survivants). Vingt-quatre heures après la première administration, on vérifie, chez tous les animaux, sous anesthésie au pentobarbital, l'efficacité de la ligature par section des carotides en aval de celle-ci. On note le nombre d'animaux survivants tant parmi les animaux traités que parmi les animaux témoins. Pour chaque dose de produit testé, le nombre d'animaux survivants parmi ceux traités par le produit est compare au nombre d'animaux survivants parmi les animaux témoins. La différence exprime l'activité de protection du produit envers la létalité induite par la ligature simultanée des deux carotides. La signification statistique (P) de cette différence est évaluée par le test de Brandt-Snedecor.

c. Résultats

Dans le tableau II ci-dessous, on donne les résultats obtenus pour des doses croissantes des produits A et B:

Tableau II

| Produit testé | Dose ip en mmol/kg | Nombre d'animaux survivants | | P |
|---|---|---|---|---|
| | | témoins | traités | |
| A | 0,32 | 6/29 | 8/29 | NS |
| | 0,64 | 11/30 | 21/30 | 0,01 |
| B | 0,1 | 9/29 | 14/29 | NS |
| | 0,16 | 6/29 | 14/30 | 0,05 |
| | 0,32 | 8/30 | 19/29 | 0,01 |

NS = différence non significative.

d. Conclusions

Le tableau II montre que le racémate (produit A) n'est actif qu'à partir d'une dose de 0,64 mmol/kg. Par contre, l'énantiomère lévogyre de l'invention (produit B) protège les animaux contre la létalité induite par la ligature simultanée des deux carotides dès 0,16 mmol/kg et s'avère donc 4 fois plus actif que le racémate.

III. Toxicité

Dans le tableau III ci-dessous, on donne, pour les produits A et B, les DL 50 par voie intraveineuse et en mg/kg, déterminées chez la souris mâle et le rat mâle:

Tableau III

| Produit testé | DL 50 en mg/kg | |
|---|---|---|
| | souris | rat |
| A | 1790 | 1500 |
| B | 1081 | 1038 |

Il ressort de ce tableau que l'énantiomère lévogyre de l'invention (produit B) est, comme le racémate (produit A) très peu toxique et que la dose toxique se situe bien au-dessus de la dose active. Le composé de la présente invention est administrable soit par voie orale sous forme de compositions solides ou liquides, par exemple sous forme de comprimés, gélules, dragées, capsules en gélatine, solutions ou sirops, soit par voie parentérale sous forme de solutions ou suspensions injectables.

Les formes pharmaceutiques telles que solutions ou comprimés sont préparées selon les méthodes couramment utilisées par les pharmaciens. On mélange le composé de l'invention avec un véhicule solide ou liquide, non toxique, pharmaceutiquement acceptable et éventuellement avec un agent dispersant, un agent stabilisant et le cas échéant, avec des agents colorants, édulcorants, etc. Les excipients pharmaceutiques solides pour la préparation de comprimés ou de capsules sont, par exemple, l'amidon, le talc, le carbonate de calcium, le lactose, le sucrose, le stéarate de magnésium, etc.

Le pourcentage de produit actif dans les compositions pharmaceutiques peut varier dans des limites très larges selon le mode d'administration et l'état du patient, la posologie humaine pouvant varier dans ces conditions entre 250 mg à 3 g par jour. A titre d'exemple non limitatif d'une composition contenant le composé de l'invention, on donne ci-après un exemple d'une gélule à 100 mg utilisable per os:

| | |
|---|---|
| produit B | 100 mg |
| avicel* | 217 mg |
| stéarate de Mg | 6 mg |

*Avicel est une marque commercial pour une cellulose microcristalline.

**Revendications pour les Etats contractants: BE, CH, DE, FR, GB, IT, LI, LU, NL, SE**

1. (S)-alpha-éthyl-2-oxo-1-pyrrolidineacétamide.

2. Procédé de préparation du (S)-alpha-éthyl-2-oxo-1-pyrrolidineacétamide, caractérisé en ce qu'on fait réagir, à une température comprise entre −10°C et −60°C, l'acide (S)-alpha-éthyl-2-oxo-1-pyrrolidineacétique successivement avec (1) un halogénoformiate d'alkyle de formule HalCOOZ dans laquelle Hal représente un atome h'halogène et Z un radical alkyle ayant 1 à 4 atomes de carbone, et (2) de l'ammoniac.

3. Procédé de préparation du (S)-alpha-éthyl-2-oxo-1-pyrrolidineacétamide, caractérisé en ce qu'on cyclise, dans un solvant inerte et en présence d'un catalyseur basique choisi parmi la 2-hydroxypyridine ou le bromure de tétrabutylammonium en présence d'hydroxyde de potassium, un (S)-2-amino-butanamide de formule

$$X\text{-}CH_2CH_2\text{-}Y\text{-}NHCH(C_2H_5)CONH_2 \quad (A)$$

dans laquelle X représente un radical ZOOC– ou $HalCH_2$–, Z étant un radical alkyle ayant 1 à 4 atomes de carbone et Hal un atome d'halogène, et Y représente un radical $-CH_2-$ ou –CO–, avec la restriction que lorsque X représente un radical ZOOC–, Y est un radical $-CH_2-$ et que lorsque X représente un radical $HalCH_2$–, Y est un radical –CO–.

4. Procédé selon la revendication 3, caractérisé en ce que le composé de formule A est un (S)-4-[[1-(aminocarbonyl)propyl]amino]-butyrate d'alkyle de formule $ZOOCCH_2CH_2CH_2NHCH(C_2H_5)CONH_2$ et en ce qu'il est préparé par condensation de (S)-2-amino-butanamide avec un 4-halogénobutyrate d'alkyle de formule $ZOOCCH_2CH_2CH_2Hal$, Z ayant la signification donnée à la revendication 3 et Hal étant un atome d'halogène.

5. Procédé selon la revendication 3, caractérisé en ce que le composé de formule A est un (S)-N-[1-(aminocarbonyl)propyl]-4-halogénobutanamide de formule $HalCH_2CH_2CH_2CONHCH(C_2H_5)CONH_2$ et en ce qu'il est préparé par condensation de (S)-2-amino-butanamide avec un halogénure de 4-halogénobutyryle de formule $HalCH_2CH_2CH_2COHal$, Hal étant un atome d'halogène.

6. Compositions thérapeutiques renfermant une quantité thérapeutiquement efficace de (S)-alpha-éthyl-2-oxo-1-pyrrolidineacétamide en association avec des excipients pharmaceutiques solides ou liquides.

**Revendications pour l'Etat contractant: AT**

1. Procédé de préparation du (S)-alpha-éthyl-2-oxo-1-pyrrolidineacétamide, caractérisé en ce que:

a. on fait réagir à une température comprise entre −10°C et −60°C l'acide (S)-alpha-éthyl-2-oxo-1-pyrrolidineacétique successivement avec (1) un halogénoformiate d'alkyle de formule HalCOOZ dans laquelle Hal représente un atome d'halogène et Z un radical alkyle ayant 1 à 4 atomes de carbone, et (2) de l'ammoniac; ou

b. on cyclise dans un solvant inerte et en présence d'un catalyseur basique choisi parmi la 2-hydroxypyridine ou le bromure de tétrabutyl ammonium en présence d'hydroxyde de potassium, un (S)-2-amino-butanamide de formule

$X-CH_2CH_2-Y-NHCH(C_2H_5)CONH_2$ (A)

dans laquelle X représente un radical ZOOC– ou $HalCH_2$–, Z étant un radical alkyle ayant 1 à 4 atomes de carbone et Hal un atome d'halogène, et

Y représente un radical $-CH_2-$ ou –CO–, avec la restriction que lorsque X représente un radical ZOOC–, Y est un radical $-CH_2-$ et que lorsque X représente un radical $HalCH_2$–, Y est un radical –CO–.

2. Procédé selon la revendication 1, caractérisé en ce que le composé de formule A est un (S)-4-[[1-(aminocarbonyl)propyl]amino]-butyrate d'alkyle de formule $ZOOCCH_2CH_2CH_2NHCH(C_2H_5)CONH_2$ et en ce qu'il est préparé par condensation de (S)-2-amino-butanamide avec un 4-halogénobutyrate d'alkyle de formule $ZOOCCH_2CH_2CH_2Hal$, Z étant un radical alkyle ayant 1 à 4 atomes de carbone et Hal un atome d'halogène.

3. Procédé selon la revendication 1, caractérisé en ce que le composé de formule A est un (S)-N-[1-(aminocarbonyl)propyl]-4-halogénobutanamide de formule $HalCH_2CH_2CH_2CONHCH(C_2H_5)CONH_2$ et en ce qu'il est préparé par condensation de (S)-2-amino-butanamide avec un halogénure de 4-halogénobutyryle de formule $HalCH_2CH_2CH_2COHal$, Hal étant un atome d'halogène.

**Patentansprüche für die Vertragsstaaten: BE, CH, DE, FR, GB, IT, LI, LU, NL und SE**

1. (S)-Alpha-äthyl-2-oxo-1-pyrrolidinacetamid.

2. Verfahren zur Herstellung von (S)-Alpha-äthyl-2-oxo-1-pyrrolidinacetamid, dadurch gekennzeichnet, dass man (S)-Alpha-äthyl-2-oxo-1-pyrrolidinessigsäure nacheinander mit (1) einem Alkylhalogenformiat der Formel HalCOOZ, worin Hal ein Halogenatom und Z einen Alkylrest mit 1 bis 4 Kohlenstoffatomen darstellt, und (2) Ammoniak, bei einer Temperatur zwischen −10°C und −60°C, umsetzt.

3. Verfahren zur Herstellung von (S)-Alpha-äthyl-2-oxo-1-pyrrolidinacetamid, dadurch gekennzeichnet, dass man ein (S)-2-Amino-butanamid der Formel

$X-CH_2CH_2-Y-NHCH(C_2H_5)CONH_2$ (A)

worin X den Rest ZOOC– oder $HalCH_2$– darstellt, wobei Z ein Alkylrest mit 1 bis 4 Kohlenstoffatomen und Hal ein Halogenatom ist, und

Y den Rest $-CH_2-$ oder –CO– darstellt,

mit der Einschränkung, dass Y der Rest $-CH_2-$ ist, wenn X den Rest ZOOC– darstellt und dass Y der Rest –CO– ist, wenn X den Rest $HalCH_2$– darstellt, in einem inerten Lösungsmittel und in Gegenwart eines basischen Katalysators ausgewählt aus 2-Hydroxypyridin oder Tetrabutylammoniumbromid in Gegenwart von Kaliumhydroxid, cyclisiert.

4. Verfahren nach Anspruch 3, dadurch gekennzeichnet, dass die Verbindung der Formel A ein Alkyl-(S)-4-[[1-(aminocarbonyl)propyl]amino]-butyrat der Formel $ZOOCCH_2CH_2CH_2NHCH(C_2H_5)CONH_2$ ist und dass sie durch Kondensation von (S)-2-Amino-butanamid mit einem Alkyl-4-halogenbutyrat der Formel $ZOOCCH_2CH_2CH_2$-Hal, wobei Z die in Anspruch 3 angegebene Bedeutung hat und Ha ein Halogenatom ist, hergestellt ist.

5. Verfahren nach Anspruch 3, dadurch gekennzeichnet, dass der Verbindung der Formel A ein (S)-N-[1-(aminocarbonyl)propyl]-4-halogenbutanamid ist und dass sie durch Kondensation von (S)-2-Amino-butanamid mit einem 4-Halogenbutyrylhalogenid der Formel $HalCH_2CH_2CH_2COHal$, wobei Hal ein Halogenatom ist, hergestellt ist.

6. Heilmittel, enthalten eine therapeutisch wirksame Menge eines (S)-Alpha-äthyl-2-oxo-1-pyrrolidinacetamids in Verbindung mit festen oder flüssigen, pharmazeutischen Hilfsstoffen.

**Patentansprüche für den Vertragsstaat: AT**

1. Verfahren zur Herstellung von (S)-Alpha-äthyl-2-oxo-1-pyrrolidinacetamid, dadurch gekennzeichnet, dass man

a. (S)-Alpha-äthyl-2-oxo-1-pyrrolidinessigsäure nacheinander mit (1) einem Alkyl-halogenformiat der Formel HalCOOZ worin Hal ein Halogenatom und Z einen Alkylrest mit 1 bis 4 Kohlenstoffatomen darstellt, und (2) Ammoniak, bei einer Temperatur zwischen −10°C und −60°C, umsetzt; oder

b. ein (S)-2-Amino-butanamid der Formel

$X-CH_2CH_2-Y-NHCH(C_2H_5)CONH_2$ (A)

worin X den Rest ZOOC– oder $HalCH_2$– darstellt, wobei Z ein Alkylrest mit 1 bis 4 Kohlenstoffatomen und Hal ein Halogenatom ist, und

Y den Rest $-CH_2-$ oder –CO– darstellt, mit der Einschränkung, dass Y der Rest $-CH_2-$ ist, wenn X den Rest ZOOC– darstellt und dass Y der Rest –CO– ist, wenn X den Rest $HalCH_2$–darstellt, in einem inerten Lösungsmittel und in Gegenwart eines basischen Katalysators ausgewählt aus 2-Hydroxypyridin oder Tetrabutylammoniumbromid in Gegenwart von Kaliumhydroxid, cyclisiert.

2. Verfahren nach Anspruch 1, dadurch gekennzeichnet, dass die Verbindung der Formel A ein Alkyl-(S)-4-[[1-(aminocarbonyl)propyl]amino]-butyrat der Formel $ZOOCCH_2CH_2CH_2NHCH(C_2H_5)CONH_2$ ist und dass sie durch Kondensation von (S)-2-Amino-butanamid mit einem Alkyl-4-halogenbutyrat der Formel $ZOOCCH_2CH_2CH_2Hal$, wobei Z die in Anspruch 1 angegebene Bedeutung hat und Hal ein Halogenatom ist, hergestellt ist.

3. Verfahren nach Anspruch 1, dadurch gekennzeichnet, dass der Verbindung der Formel A ein (S)-N-[1-(aminocarbonyl)propyl]-4-halogenbutanamid ist und dass sie durch Kondensation von (S)-2-Amino-butanamid mit einem 4-Halogenbutyrylhalogenid der Formel $HalCH_2CH_2CH_2COHal$, wobei Hal ein Halogenatom ist, hergestellt ist.

**Claims for the Contracting States: BE, CH, DE, FR, GB, IT, LI, LU, NL and SE.**

1. (S)-alpha-ethyl-2-oxo-1-pyrrolidineacetamide.

2. Process for the preparation of (S)-alpha-ethyl-2-oxo-1-pyrrolidineacetamide, characterised in that (S)-alpha-ethyl-2-oxo-1-pyrrolidineacetic acid is reacted, at a temperature between −10°C and −60°C, successively with (1) an alkyl haloformate of the formula HalCOOZ in which Hal represents a halogen atom and Z an alkyl radical having 1 to 4 carbon atoms, and with (2) ammonia.

3. Process for the preparation of (S)-alpha-ethyl-2-oxo-1-pyrrolidineacetamide, characterised in that an (S)-2-amino-butanamide of the formula

$$X\text{-}CH_2CH_2\text{-}Y\text{-}NHCH(C_2H_5)CONH_2 \qquad (A)$$

in which X represents a ZOOC– or $HalCH_2$– radical, Z being an alkyl radical having 1 to 4 carbon atoms an Hal a halogen atom, and
Y represents a $-CH_2-$ or –CO– radical, with the proviso that Y is a $-CH_2-$ radical when X represents a ZOOC– radical and Y is a –CO– when X represents a $HalCH_2$– radical, is cyclised in an inert solvent and in the presence of a basic catalyst selected from 2-hydroxypyridine or tetrabutylammonium bromide in the presence of potassium hydroxide.

4. Process according to claim 3, characterised in that the compound of formula A is an alkyl (S)-4-[[1-(aminocarbonyl)propyl]amino]-butyrate of the formula $ZOOCCH_2CH_2CH_2NHCH(C_2H_5)CONH_2$ and that it is prepared by condensing (S)-2-amino-butanamide with an alkyl 4-halobutyrate of the formula $ZOOCCH_2CH_2CH_2Hal$, Z having the meaning given in claim 3 and Hal being a halogen atom.

5. Process according to claim 3, characterised in that the compound of formula A is an (S)-N-[1-(aminocarbonyl)propyl]-4-halobutanamide of the formula $HalCH_2CH_2CH_2CONH(C_2H_5)CONH_2$ and that it is prepared by condensing (S)-2-amino-butanamide with a 4-halobutyryl halide of the formula $HalCH_2CH_2CH_2COHal$, Hal being a halogen atom.

6. Therapeutic compositions containing a therapeutically effective amount of (S)-alpha-ethyl-2-oxo-1-pyrrolidineacetamide in association with solid or liquid pharmaceutical excipients.

**Claims for the Contracting State: AT**

1. Process for the preparation of (S)-alpha-ethyl-2-oxo-1-pyrrolidineacetamide, characterised in that:
a. (S)-alpha-ethyl-2-oxo-1-pyrrolidineacetic acid is reacted at a temperature between -10°C and -60°C, successively with (1) an alkyl haloformate of the formula HalCOOZ in which Hal represents a halogen atom and Z an alkyl radical having 1 to 4 carbon atoms, and with (2) ammonia; or
b. an (S)-2-amino-butanamide of the formula

$$X\text{-}CH_2CH_2\text{-}Y\text{-}NHCH(C_2H_5)CONH_2 \qquad (A)$$

in which X represents a ZOOC– or $HalCH_2$– radical, Z being an alkyl radical having 1 to 4 carbon atoms and Hal a halogen atom, and
Y represents a $-CH_2-$ or –CO– radical, with the proviso that Y is a $-CH_2-$ radical when X represents a ZOOC– radical and Y is a –CO– radical when X represents a $HalCH_2$– radical, is cyclised in an inert solvent and in the presence of a basic catalyst selected from 2-hydroxypyridine or tetrabutylammonium bromide in the presence of potassium hydroxide.

2. Process according to claim 1, characterised in that the compound of formula A is an alkyl (S)-4-[[1-(aminocarbonyl)propyl]amino]-butyrate of the formula $ZOOCCH_2CH_2CH_2NHCH(C_2H_5)CONH_2$ and that it is prepared by condensing (S)-2-amino-butanamide with an alkyl 4-halobutyrate of the formula $ZOOCCH_2CH_2CH_2Hal$, Z being an alkyl radical having 1 to 4 carbon atoms and Hal a halogen atom.

3. Process according to claim 1, characterised in that the compound of formula A is an (S)-N-[1-(aminocarbonyl)propyl]-4-halobutanamide of the formula $HalCH_2CH_2CH_2CONH(C_2H_5)CONH_2$ and that it is prepared by condensing (S)-2-amino-butanamide with a halobutyryl halide of the formula $HalCH_2CH_2CH_2COHal$, Hal being a halogen atom.